# FASCICULE DE BREVET EUROPEEN

(11) **EP 2 296 733 B1**
(45) Date de publication et mention de la délivrance du brevet: **14.05.2014**
(21) Numéro de dépôt: 09784480.7
(22) Date de dépôt: 07.07.2009
(51) Int. Cl.: A61M 5/24, A61M 5/315

(54) **DISPOSITIF D'INJECTION D'UN FLUIDE A USAGE MEDICAL**
VORRICHTUNG ZUR INJEKTION EINER MEDIZINISCHEN FLÜSSIGKEIT ZUR MEDIZINISCHEN VERWENDUNG
DEVICE FOR INJECTING FLUID FOR MEDICAL USE

(30) Priorité: 07.07.2008 FR 0803856
(43) Date de publication de la demande: 23.03.2011
(73) Titulaire: Cahen, Jean-Paul, 38330 Montbonnot (FR)
(72) Inventeur: Cahen, Jean-Paul, 38330 Montbonnot (FR)
(74) Mandataire: Gasquet, Denis
(86) Numéro de dépôt international: PCT/FR2009/051346
(87) Numéro de publication internationale: WO 2010/004206

(56) Documents cités:
- EP-A- 1 086 661
- FR-A- 1 288 915
- GB-A- 2 214 819
- US-A- 5 147 311
- US-A- 5 378 233
- US-A1- 2002 091 361
- US-B1- 6 332 876

## Description

L'invention concerne un dispositif d'injection d'un fluide à usage médical destiné au corps humain ou animal.

La médecine actuelle utilise différents types d'injection permettant d'introduire dans le corps des patients un produit à diverses fins. Citons, par exemple, les transfusions ou les procédés de nutrition artificielle, notamment par le sang ou par voie digestive (parentérale ou entérale). Les procédés d'analyse et de contrôle de l'état des patients utilisent aussi des injections de produits de contraste. C'est le cas, par exemple, des analyses du type angiographie pour lesquelles on injecte un produit iodé dans les vaisseaux, veines et artères pour opacifier ceux-ci ainsi que les tissus et organes irrigués afin de déceler, par radio, les anomalies éventuelles. On utilise également des injections de produits de contraste iodés pour réaliser un scanner ou une angiographie traditionnelle ou numérisée, l'injection veineuse ou artérielle devant se faire sous forme d'un bolus à vitesse allant généralement de 0,5 à 35 ml/s.

Les examens IRM (Résonance Magnétique Nucléaire) nécessitent également l'injection d'un produit de contraste (Gadolinium par exemple).

On connaît divers dispositifs d'injection. Ceux-ci sont généralement constitués d'un d'injecteur contenant le produit à injecter actionné par un mécanisme, électro-mécanique ou hydraulique. Les injecteurs utilisés sont, par exemple, à poche souple où le produit est conditionné directement dans des enveloppes souples. Ces enveloppes sont ensuite disposées dans une enceinte fermée du dispositif d'injection mise sous pression, voir par ex. EP 1 086 661 A2. Le préambule de la revendication 1 est basé sur ce document.

On utilise aussi des injecteurs du type seringue où le produit est conditionné dans le corps de la seringue qui comprend un piston mobile actionné manuellement ou mécaniquement grâce à un dispositif d'injection dont le mécanisme actionne en translation axiale le piston.

Les injecteurs à poche souple présentent l'avantage de pouvoir être remplis à l'avance du produit à injecter, ce qui facilite les manipulations et permet une meilleure asepsie. Toutefois, l'éjection du produit nécessitant une enceinte de mise en pression, cela augmente l'encombrement et le poids des dispositifs d'injection ainsi que leur manipulation.

Les injecteurs à seringue sont d'une mise en oeuvre plus simple mais ils présentent l'inconvénient de ne pouvoir être remplis qu'au moment de leur utilisation. De sorte que leur manipulation, avant d'être placés dans le dispositif d'injection est plus longue et surtout peut présenter plus de risque de contamination.

Le but de l'invention est donc de proposer un injecteur qui combine les avantages des deux types précités, sans en avoir les inconvénients.

A cet effet, la présente invention a pour objet un dispositif d'injection d'un fluide à usage médical comportant un fourreau dans lequel est installé un réservoir destiné à recevoir le fluide à injecter, constitué d'un corps déformable axialement, d'une face supérieure munie d'un embout de passage du fluide engagé dans un aménagement approprié du sommet de la gaine et d'un fond venant en appui sur un piston mobile en translation axiale dans la gaine.

Le réservoir de l'invention est donc une seringue déformable sans piston, puisque sa déformation d'une position déployée vers une position repliée, se fait par déformation de sa paroi, qui est constituée par une succession d'anneaux circulaires déformables, aptes à se replier sur eux-même.

Selon d'autres caractéristiques avantageuses de l'invention :
- La paroi interne du sommet de la gaine et la paroi externe de la face supérieure du réservoir ont des formes complémentaires.
- De même, la paroi externe du fond du réservoir et le piston ont des formes complémentaires.
- De préférence, la face supérieure et le fond du réservoir ont une forme conique ajustée pour permettre l'emboîtement dudit fond dans la face supérieure à la fin du mouvement de translation axiale du piston.
- Le volume engendré par la surface conique du fond du réservoir forme un logement externe en creux dans lequel vient s'emboîter la forme complémentaire du piston.
- Le fond du réservoir comporte en son centre un téton aligné axialement avec l'embout porté par la face supérieure et dont le diamètre est sensiblement égal, au jeu fonctionnel près, au diamètre intérieur de celui-ci. Il résulte de cette géométrie complémentaire un volume résiduel nul en fin d'injection.
- Le fourreau comporte une fenêtre pour le passage de réservoir et son sommet est pourvu d'une encoche débouchant, d'une part dans la fenêtre et d'autre part dans une ouverture centrale du sommet, de dimensions appropriées pour recevoir et maintenir l'embout du réservoir.
- La paroi du corps du réservoir est en forme de soufflet dont le profil autorise un pliage lors de la compression.
- En variante de réalisation, la paroi du corps du réservoir est formée d'un soufflet à anneaux circulaires.
- Avantageusement, la rigidité latérale de la structure du réservoir est ajustée pour empêcher toute élasticité parasite.
- La géométrie du soufflet est telle que les déformations autres que celles générées par la translation axiale sont quasi nulles.
- Pour permettre le remplissage du réservoir, le piston est muni d'un dispositif permettant de le solidariser au fond dudit réservoir lorsque celui-ci est vide et il est animé d'un mouvement de translation axiale de traction de celui-ci.
- Dans un mode de réalisation, le fourreau peut être doublé de façon à recevoir plusieurs réservoirs, la commande de chacun du réservoir se faisant de manière indépendante.

D'autres caractéristiques et avantages de l'invention apparaîtront clairement à la lecture de la description ci-après, donnée à titre indicatif en référence aux dessins annexés dans lesquels :
- La figure 1 est une vue en perspective en coupe du réservoir selon l'invention, en position déployée,
- La figure 2 est une vue en perspective avec une coupe du même réservoir en position repliée,
- Les figures 3 et 4 sont des vues analogues aux précédentes figures mais selon des vues extérieures sans arrachement.
- La figure 5 est une vue en perspective du dispositif d'injection, en cours d'utilisation.
- La figure 6 est une vue en perspective du dispositif d'injection, en fin d'injection, le réservoir étant en position complètement repliée.
- La figure 7 est une vue du dispositif d'injection montrant les manipulations du réservoir.
- La figure 8 est une vue de détail d'un perfectionnement.

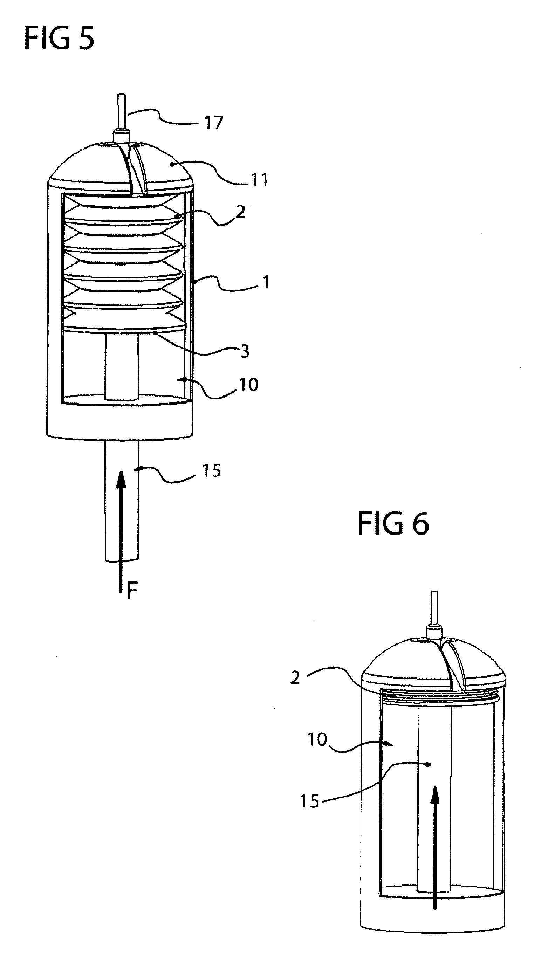

Le dispositif d'injection selon l'invention est constitué (figure 5) d'un support (1) qui selon le mode de réalisation illustré est un fourreau de forme générale cylindrique, qui porte un réservoir (2) destiné à recevoir le fluide à injecter. Le support (1) comprend un piston (3) mobile en translation axiale.

Le réservoir (2) est déformable et est réalisé dans un matériau déformable tel que par exemple en matière plastique, par exemple en polypropylène, autorisant son remplissage préalable par le fluide à injecter.

Ce réservoir (2) forme un volume dont la paroi est déformable axialement. Pour cela la paroi est constituée d'une partie supérieure (4) et d'un fond (5) tous deux ayant la forme d'un cône. La paroi périphérique formant le corps de l'enceinte (6) a la forme d'un soufflet déformable à anneaux circulaires (21) de sorte que, lorsque le soufflet est entièrement replié sur lui-même, le réservoir est totalement vide sans liquide résiduel. Ainsi la paroi du réservoir ayant la forme d'un soufflet est constituée d'une succession d'anneaux circulaires dont la périphérie est de section triangulaire chacun des anneaux comprenant ainsi une face supérieure (210) conique vers le haut et une face inférieure (211) conique vers le bas. On notera qu'en position repliée chacune des faces supérieures (210) se trouve en contact avec la face inférieure (211) de l'anneau adjacent.

On notera que la face supérieure (4) du réservoir porte en son centre un embout (7) cylindrique pour le passage du fluide contenu dans ledit réservoir.

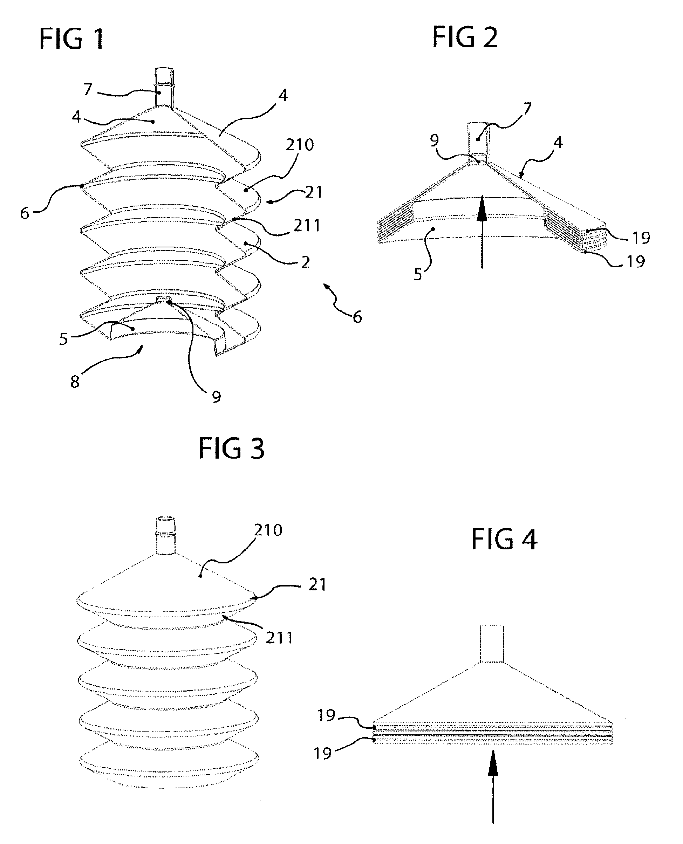

Le volume engendré par la surface conique du fond (5) se trouve à l'extérieur du volume du réservoir et forme ainsi un logement (8) en creux. Notons que le fond (5) comporte en son centre un téton de centrage (9) aligné axialement avec l'embout (7) et dont le diamètre est sensiblement égal, à la liberté de débattement axial près, au diamètre intérieur de celui-ci, de façon à ce que position repliée ledit téton se trouve engagé dans l'embout comme cela est illustré à la figure 2.

Le support (1) comporte une ouverture latérale (10) de dimensions suffisantes formant une fenêtre pour permettre le passage et la mise en place du réservoir (2), tel qu'illustré à la figure 5. Selon le mode de réalisation illustré selon lequel le support est un fourreau, ce dernier est fermé, en partie supérieure par un sommet (11) en forme de calotte sphérique à l'extérieur et dont les parois intérieures forment une surface conique dont la forme est complémentaire de la paroi extérieure de la partie supérieure (4) du réservoir.

Le sommet (11) comporte une encoche radiale (12) débouchant, d'une part dans la fenêtre (10) et d'autre part dans une ouverture centrale (13) du sommet.

Dans le fourreau (1) est logé un piston (3) solidaire d'une extrémité d'un axe (15) qui traverse la base (16) du fourreau pour être relié par sa deuxième extrémité à un mécanisme d'actionnement propre à engendrer un mouvement de translation axiale F transmis par l'axe (15) au piston (3).

Selon un mode de réalisation de l'invention, la forme extérieure du piston est complémentaire de celle de la surface en vis-à-vis du logement (8) formé dans le fond (5) du réservoir.

On notera que le support (1) qui a la forme d'un fourreau selon les illustrations proposées à titre d'exemple, pourrait avoir une toute autre forme. Ainsi, il pourrait ne pas avoir de paroi périphérique, et ainsi être constitué par un étrier comprenant une paroi supérieure constituant le sommet (11) relié à une paroi inférieure constituant la base (16) par un ou plusieurs bras de liaison, voire toute autre liaison.

Le procédé de mise en place du réservoir dans le support (1) se déduit aisément de la description précédente. Le réservoir (2) à l'intérieur duquel se trouve le fluide médical y est introduit, à travers la fenêtre (10) selon un mouvement de translation transversale. Dans cette étape, le piston (3) est placé en position basse où il repose sur la base (16) du support.

L'embout (7) du réservoir, muni d'une canule (17) fixée sur l'embout au moyen d'une connexion (18) est introduit, par l'encoche (12), dans l'ouverture (13) de la calotte (11). Il peut être prévu des moyens de blocage (non représentés), destinés à empêcher le pivotement du réservoir autour de son axe.

La forme du piston (3) qui est de forme complémentaire au logement en creux (8) du fond (5) du réservoir (2), s'emboîte parfaitement l'un dans l'autre, venant ainsi bloquer celui-ci en déplacement transversal.

Lorsque le mécanisme d'actionnement exerce sur l'axe (15) du piston un mouvement de translation selon (F) suivant l'axe longitudinal, ledit piston se déplace dans le même sens en exerçant sur le réservoir une force de pression qui engendre la contraction, donc le pliage et la contraction de la paroi (6) déformable du réservoir et, dans le même temps le refoulement du fluide que contient celui-ci.

On notera que la complémentarité de la forme du piston et du fond du réservoir permet un pliage régulier du corps du réservoir, anneaux de pliage (19) s'emboîtant les uns dans les autres, tel que cela est illustré aux figures 2 et 4.

A la fin de l'expulsion du fluide, lorsque le réservoir est complètement contracté, le fond (5) du réservoir est au contact de la face supérieure (4) de celui-ci et son téton (9) est engagé dans l'embout (7) de façon à ne laisser aucune quantité de fluide résiduel. Celui-ci est, en fait, proche de zéro, en raison de la forme adaptée de chacun des éléments coopérants qui permet d'obtenir, en fin de translation des anneaux de pliage 19 parfaitement jointifs.

Le réservoir vide est ensuite retiré de la gaine selon un mouvement de translation inverse de celui ayant permis son introduction.

On notera que la forme conique du réservoir empêche l'accrochage sur ses parois de bulles d'air pouvant se former lors de son remplissage. Celles-ci se localisent donc au sommet et sont évacuées en priorité lors de l'opération de débullage.

On précisera que la rigidité latérale de la structure du réservoir est ajustée pour éviter toute élasticité parasite dans la limite des pressions courantes pour ce type de matériel (21 bars, 300 psi) et que le réservoir est étroitement maintenu dans sa gaine, au jeu de débattement axial près.

En effet, la poussée exercée sur le réservoir par le piston doit générer un avancement continu. Pour ce faire, il est nécessaire que la déformation du réservoir s'exerce uniquement dans le sens axial, c'est-à-dire dans la direction de la force F engendrée par le piston afin de maîtriser le volume instantané et, par conséquent, le débit et la pression d'éjection du fluide contenu dans ledit réservoir.

Le mécanisme d'actionnement du piston est choisi, en conséquence, pour générer un mouvement de translation continu et réglable, par exemple, un vérin hydraulique ou un moteur électrique, notamment à contrôle électronique sans balai.

Une fois vide, le réservoir peut être à nouveau rempli. Pour cela on utilise la gaine que l'on vient de décrire ou une gaine analogue. Le réservoir est placé dans la position représentée figure 6 dans laquelle il est plaqué contre la paroi intérieure du sommet (11) de la gaine, son embout (7) étant maintenu dans l'ouverture (13) de celui-ci.

Le piston est muni d'un dispositif permettant de le solidariser au fond du réservoir et son mécanisme d'actionnement exerce sur lui un mouvement de translation axiale en sens inverse du précédent (F). Le fond (5) du réservoir est muni d'un moyen d'accrochage au piston (3) permettant ainsi un remplissage par aspiration de la même manière qu'une seringue à piston traditionnelle. Le fond (5) du réservoir est entraîné vers la base du fourreau par le piston afin que le réservoir reprenne progressivement sa forme initiale tout en aspirant le liquide.

On ajoutera encore que la gaine peut être adaptée de façon à recevoir plusieurs réservoirs, la commande de chacun du réservoir se faisant de manière indépendante (plusieurs fourreaux).

Le dispositif d'injection que l'on vient de décrire permet, avec le même système d'injection, soit d'utiliser des réservoirs remplis au préalable du fluide à injecter, soit d'effectuer ce remplissage au moment de son utilisation.

Il est donc particulièrement souple et polyvalent. En outre sa mise en place et son système d'injection sont simples et il ne nécessite pas de générateur de puissance pénalisant du point de vue coût et encombrement.

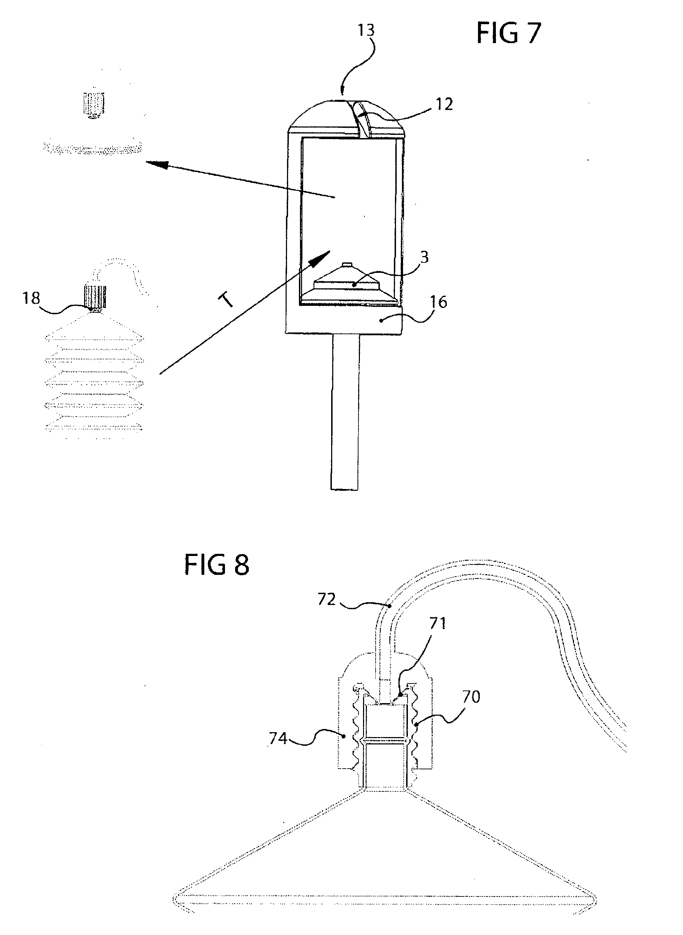

La figure 8 est une vue de détail d'un perfectionnement selon lequel l'embout (7) comprend un bouchon (70) équipé d'un opercule (71), tandis que lors de la connexion au tuyau d'injection (72), une saillie (73) du connecteur (74) perfore l'opercule (711).

On a compris que lorsque le réservoir (2) lorsqu'il est complètement comprimé en fin d'injection, est tel que les anneaux constituant le soufflet sont jointifs avec un emboîtement parfait de façon à ce qu'il ne reste aucun liquide médical résiduel

On a compris aussi que la paroi de fond (16) sert de guidage pour la tige de piston, tandis que la paroi du sommet (11) sert d'appui pour l'enceinte (2).

## Revendications

1. Dispositif d'injection d'un fluide à usage médical, constitué d'un support (1) dans lequel est installé un réservoir (2) destiné à recevoir le fluide à injecter, ledit réservoir étant formé d'un corps (6) réalisé en matériau déformable, ayant la forme d'un soufflet pour pouvoir être comprimé axialement et comprenant une face supérieure (4) comportant un embout (7) de passage du fluide, tandis que le support (1) est constitué par une paroi de fond (16) permettant le guidage de la tige d'un piston (3) mobile en translation axiale, et une paroi de sommet (11),
- l'embout (7) est engagé dans un aménagement approprié (12), (13) de la paroi de sommet (11) du support, **caractérisé en ce que**
- la face supérieure (4) et la paroi de fond (5) ont la forme d'un cône, tandis que l'enceinte (6) du réservoir (2) est constitué d'anneaux circulaires, et que le piston (3) est de forme complémentaire à la forme du logement (8) de la paroi de fond (5) de sorte que lorsque le soufflet est entièrement replié sur lui-même après injection l'enceinte est vide sans liquide résiduel,
et **en ce que** le fond (5) comporte en son centre un téton (9) aligné axialement avec l'embout (7) porté par la face supérieure (4) et dont le diamètre est sensiblement égal, à la liberté de débattement axial près, au diamètre intérieur de celui-ci.

2. Dispositif d'injection selon la revendication 1
**caractérisé en ce que** la paroi du réservoir est constituée d'une succession d'anneaux circulaires dont la périphérie est de section triangulaire chacun des anneaux comprenant ainsi une face supérieure (210) conique vers le haut et une face inférieure (211) conique vers le bas, de façon à ce qu'en position repliée chacune des faces supérieures (210) se trouve en contact avec la face inférieure (211) de l'anneau adjacent.

3. Dispositif d'injection selon la revendications 2, **caractérisé en ce que** l'enceinte (2) est réalisée en matière plastique.

4. Dispositif d'injection selon l'une des revendications précédentes,
**caractérisé en ce que** le support (1) comporte une fenêtre (10) pour le passage de réservoir et **en ce que** son sommet (11) est pourvu d'une encoche (12) débouchant, d'une part dans la fenêtre (10) et d'autre part dans une ouverture centrale (13) du sommet de dimensions appropriées pour recevoir et maintenir l'embout (7) du réservoir.

5. Dispositif d'injection selon l'une quelconque des revendications précédentes,
**caractérisé en ce que** le piston (3) est muni d'un dispositif permettant de le solidariser au fond (5) du réservoir lorsque celui-ci est vide et **en ce qu'**il est animé d'un mouvement de translation axiale de traction de celui-ci.

6. Dispositif d'injection selon l'une des revendications précédentes,
**caractérisé en ce que** le support est adaptée de façon à recevoir plusieurs réservoirs, la commande de chacun du réservoir se faisant de manière indépendante.

7. Dispositif d'injection selon l'une des revendications précédentes
**caractérisé en ce que** les réservoirs prêts à l'emploi, vides ou pleins, sont équipés d'embout percutable garantissant la stérilité de l'ensemble.

## Patentansprüche

1. Vorrichtung zur Injektion eines Fluids für medizinische Zwecke, bestehend aus einem Träger (1) in dem ein Behälter (2) zur Aufnahme des zu injizierenden Fluids eingesetzt ist, wobei der Behälter aus einem Körper (6) gebildet ist, der aus einem verformbaren Werkstoff ausgebildet ist, eine Balgform aufweist, so dass er axial komprimiert werden kann, und eine obere Seite (4) umfasst, die ein Ansatzstück (7) für den Durchlauf des Fluids aufweist, während der Träger (1) durch eine Bodenwand (16), die eine Führung einer axial verfahrbaren Stange eines Kolbens (3) erlaubt, und eine Deckwand (11) gebildet ist,
- das Ansatzstück (7) in einer geeigneten Aufnahme (12), (13) der Deckwand (11) des Trägers eingesetzt ist, **dadurch gekennzeichnet, dass**
- die obere Seite (4) und die Bodenwand (5) kegelförmig sind, während das Gehäuse (6) des Behälters (2) aus kreisförmigen Ringen besteht und dass der Kolben (3) eine der Form der Aufnahme (8) der Bodenwand (5) entsprechende Form aufweist, so dass wenn der Balg nach der Injektion völlig in sich zusammengefaltet ist, das Gehäuse ohne Restflüssigkeit entleert ist,
und dass der Boden (5) in seiner Mitte einen Stutzen (9) aufweist, der axial auf das durch die Oberseite (4) getragene Ansatzstück (7) ausgerichtet ist, und dessen Durchmesser dem inneren Durchmesser des Ansatzstückes,bis auf den axialen Ausschlag, im Wesentlichen gleicht.

2. Vorrichtung zur Injektion nach Anspruch 1,
**dadurch gekennzeichnet, dass** die Wand des Behälters aus einer Reihe von kreisförmigen Ringen gebildet ist, deren Umfang einen dreieckigen Querschnitt aufweist, wobei jeder der Ringe somit eine nach oben weisende kegelförmige obere Seite (210) und eine nach unten weisende kegelförmige untere Seite (211) aufweist, so dass in einer gefalteten Stellung jede der oberen Flächen (210) in Kontakt mit der unteren Fläche (211) des benachbarten Ringes steht.

3. Vorrichtung zur Injektion nach Anspruch 2,
**dadurch gekennzeichnet, dass** der Behälter (2) aus einem Kunststoff ausgeführt ist.

4. Vorrichtung zur Injektion nach einem der vorhergehenden Ansprüche,
**dadurch gekennzeichnet, dass** der Träger (1) ein Fenster (10) aufweist, um den Behälter passieren zu lassen, und dass dessen Scheitel (11) mit einer Nut (12) versehen ist, die einerseits in dem Fenster (10) und andererseits in einer zentralen Öffnung (13) desscheitels mündet, deren Abmessungen zur Aufnahme und Halterung des Ansatzstückes (7) des Behälters geeignet ist.

5. Vorrichtung zur Injektion nach einem der vorhergehenden Ansprüche,
**dadurch gekennzeichnet, dass** der Kolben (3) mit einer Vorrichtung ausgestattet ist, anhand derer er bei leerem Behälter mit dem Boden (5) des Behälters fest verbunden werden kann und dass er durch Ziehen axial verfahrbar ist.

6. Vorrichtung zur Injektion nach einem der vorhergehenden Ansprüche,
**dadurch gekennzeichnet, dass** der Träger zur Aufnahme mehrerer Behälter geeignet ist, wobei jeder Behälter unabhängig angesteuert wird.

7. Vorrichtung zur Injektion nach einem der vorhergehenden Ansprüche,
**dadurch gekennzeichnet, dass** die gebrauchsfertigen, leeren oder vollen Behälter mit einem anstechbaren Ansatzstück versehen sind, um die Sterilität der Einheit zu gewährleisten.

## Claims

1. Device for injecting a fluid for medical use, consisting of a support (1) in which a reservoir (2) intended to receive the fluid to be injected is installed, said reservoir being formed by a body (6) produced from a deformable material, having the form of a bellows so as to be able to be compressed axially and comprising a top face (4) comprising an end piece (7) for passage of the fluid, while the support (1) is formed by a bottom wall (16) for guiding the rod of a piston (3) which is able to move in axial translation, and a top wall (11),
- the end piece (7) is engaged in a suitable arrangement (12), (13) on the top wall (11) of the support,
**characterised in that**
- the top face (4) and the bottom wall (5) are in a cone shape, while the enclosure (6) of the reservoir (2) consists of circular rings, and **in that** the piston (3) has a shape complementary to the shape of the housing (8) of the bottom wall (5) so that, when the bellows is entirely folded on itself after injection, the enclosure is empty without any residual liquid,
and **in that** the bottom (5) comprises at its centre a stud (9) aligned axially with the end piece (7) carried by the top face (4) and the diameter of which is substantially equal, except for the freedom of axial movement, to the inside diameter of said end piece.

2. Injection device according to claim 1,
**characterised in that** the wall of the reservoir consists of a succession of circular rings, the periphery of which has a triangular cross section, each of the rings thus comprising an upwardly conical top face (210) and a downwardly conical bottom face (211), so that, in the folded position, each of the top faces (210) is in contact with the bottom face (211) of the adjacent ring.

3. Injection device according to claim 2,
**characterised in that** the enclosure (2) is produced from plastics material.

4. Injection device according to one of the preceding claims,
**characterised in that** the support (1) comprises a window (10) for the reservoir to pass and **in that** its top (11) is provided with a recess (12) emerging firstly in the window (10) and secondly in a central opening (13) in the top with dimensions suitable for receiving and holding the end piece (7) of the reservoir.

5. Injection device according to any one of the preceding claims,
**characterised in that** the piston (3) is provided with a device for securing it to the bottom (5) of the reservoir when the latter is empty and **in that** it is driven in an axial translation movement of traction thereof,

6. Injection device according to one of the preceding claims,
**characterised in that** the support is adapted so as to receive several reservoirs, the control of each reservoir taking place independently.

7. Injection device according to one of the preceding claims,
**characterised in that** the reservoirs ready for use, empty or full, are equipped with a strikable end piece guaranteeing sterility of the whole.
